# EUROPEAN PATENT SPECIFICATION

(11) **EP 2 182 963 B1**
(45) Date of publication and mention of the grant of the patent: **14.08.2013**
(21) Application number: 08762895.4
(22) Date of filing: 17.06.2008
(51) Int. Cl.: A61K 36/00, A61K 47/14, A61K 47/20, A61K 47/38, A61K 9/08, A61K 36/185, A61K 36/235, A61K 36/47, A61K 36/75

(54) **HERBAL COMPOSITION ON THE BASIS OF EXTRACTS OF FOENICULUM VULGARE, MURRAYA KOENIGII AND TRIPHALA**
PFLANZENZUSAMMENSETZUNG AUF BASIS VON EXTRAKTEN VON FOENICULUM VULGARE, MURRAYA KOENIGII UND TRIPHALA
COMPOSITION À BASE D'HERBES COMPRENANT DES EXTRAITS DE FOENICULUM VULGARE, MURRAYA KOENIGII ET TRIPHALA

(30) Priority: 19.07.2007 IN DE15302007
(43) Date of publication of application: 12.05.2010
(73) Proprietor: Delhi Institute Of Pharmaceutical Sciences And Research (DIPSAR), New Dehli 110 017 (IN); Promed Exports Private Limited, New Delhi 110 016 (IN)
(72) Inventor: GUPTA, Suresh, Kumar, Pushp Vihar New Dehli 110 017 (IN); AGRAWAL, Shyam, Sunder, Pushp Vihar New Dehli 110 017 (IN); SELVAN, Vivekanandan, Kalai, Pushp Vihar New Dehli 110 017 (IN); SRIVASTAVA, Sushma, Pushp Vihar New Dehli 110 017 (IN); SAXENA, Rohit, Pushp Vihar New Dehli 110 017 (IN); CHAUHAN, Sateesh, Kumar, New Delhi 110 016 (IN); BAHRI, Deepak, New Delhi 110 016 (IN)
(74) Representative: Scheuermann, Erik
(86) International application number: PCT/IB2008/001569
(87) International publication number: WO 2009/010835

(56) References cited:
- WO-A1-2006/003383
- WO-A2-2005/120173
- IN-A- 552M UM2 004
- DATABASE TCM [Online] SIPO; 6 July 2005 (2005-07-06), Lei Jufang: "Tibetan medicine for treating eye diseases, and its preparing method", XP002598797, Database accession no. AN-200510080697-A & CN 1 709 446 A (GANSU QIZHENG TIBETAN MEDICINE [CN]) 21 December 2005 (2005-12-21)
- GROVER J K ET AL: "Assessment of hypoglycemic/antihyperglycemic activities of certain plants and their possible protective effect in diabetic complications in animals", MEDICINAL & AROMATIC PLANTS ABSTRACTS, SCIENTIFIC PUBLISHERS, NEW DELHI - INDIA, vol. 26, no. 1, 1 February 2004 (2004-02-01), XP018001903,
- MUHAMMAD HAMAYUN 1 ET AL: "Study on traditional knowledge and utility of medicinal herbs ofdistrict Buner, NWFP, Pakistan", INDIAN JOURNAL OF TRADITIONAL KNOWLEDGE, RESOURCES, NEW DELHI, NEW DELHI - INDIA, vol. 5, no. 3, 1 July 2006 (2006-07-01), pages 407-412, XP018021444, ISSN: 0972-5938
- DATABASE EMBASE BISWAS N.R. ET AL.: 'Evaluation of Ophthacare eye drops - A herbal formulation in the management of various ophthalmic disorders', XP009008143 Database accession no. EMB-2001386087 & PHYTOTHERAPY RESEARCH vol. 15, no. 7, 2001, pages 618 - 620
- VINUTHAN M.K. ET AL.: 'Effect of extracts of Murraya Koenigii leaves on the level of blood glucose and plasma insulin in alloxan- induced diabetic rats' INDIAN J. PHYSIOL. PHARMACOL. vol. 48, no. 3, 2004, pages 348 - 352, XP008129632
- DATABASE WPI Derwent Publications Ltd., London, GB; AN 2003-345669, XP008129712 & JP 2002 275079 A (FANCAL CORPORATION) 25 September 2002

## Description

### Field of the Invention

The present invention relates to a synergistic herbal composition for delaying the onset and progression of cataract.

### Background and prior art

Cataract is one of the leading causes of visual disability; often leading to blindness .The situation can only be remedied surgically by extirpation of the cataractous lens. However, even after surgery the disability still remains prevalent in substantial number of individuals.

Cataract is defined as opacity of the lens and loss of eye lens transparency. Nearly 19 million people are blind due to cataract in the world. The age-adjusted prevalence of cataract in India is three times that of the United States. Apart from aging, various risk factors such as nutritional deficiencies or inadequacies, diabetes, sunlight, environmental factors, smoking and lack of consumption of antioxidants are known to increase the risk of cataract. New medical advances such as surgery and lens replacement have a high degree of efficacy, but are not available or too expensive for most of the world's population. For this reason, if an intervention is able to delay the onset of cataract by 10 years or so, the number of cataract surgery can be reduced to nearly half.

The eye lens is a vascular tissue packed with protein that provides the high refractive index necessary for the fine focusing of light onto the retina. Visual impairment in cataract results from opacity or light scattering produced most often by the formation of large protein aggregates in the lens. The process of aggregation involves variety of complex metabolic and physiological mechanisms, which act in combination to change the refractive index. Studies on lens proteins indicate that post translational modifications such as oxidation, glycation, Schiffs base formation; phosphorylation, elevated calcium levels and proteolysis lead to lens opacification. These chemical changes can be regulated to maintain lens homeostasis and transparency. Thus, cataract is believed to be a disease that requires a biochemical and pharmacological, rather than a surgical, solution.

Although cataract is a multi-factorial disease, it is widely accepted that oxidative free-radical damage is an important factors which initiate development of cataract. Oxidative stress may cause direct modification of the inner lens proteins, such as crosslinking aggregation, and precipitation. Hence oxidative mechanisms through generation of reactive oxygen radicals are believed to play an important role in the progressive decline of vision and formation of cataract.

In vitro and in vivo studies with experimental animal models as well as the epidemiological studies conducted with selected human populations demonstrated a lower incidence of cataract in the groups consuming higher amount of ascorbic acid and other antioxidants. Reactive oxygen species (ROS) such as hydrogen peroxide, super- oxide radical, singlet oxygen and hydroxyl radical are postulated to contribute to this process. ROS are generated in the eye both enzymatically and photo-chemically. Endogenous defense mechanisms through scavenging of ROS by antioxidant enzymes like super oxide dismutase, glutathione peroxidase, catalase and glutathione-S-transferase, simultaneously protect the lens from oxidative damage.

Medicinal plant based compositions are being used since long for a variety of diseases. Because of sufficient evidence that oxidative stress play a role in mechanism of cataractogenesis, there has been an increasing interest in the development of suitable antioxidant products of plant origin that could be effective in delaying or preventing the formation of cataract. Flavonoids and related poly-phenols are antioxidants and also potent inhibitors of aldose reductase activity, the key enzyme in diabetic cataract. Further a decreased risk of cataract has been associated with consumption of tea, a major source of flavonoid- quercetin. Quercetin has been shown to inhibit hydrogen peroxide-induced oxidation of the lens proteins. Optimization of dietary intake of protective nutrients has been an effective approach towards reducing the incidence of cataract.

Curcumin, the active principle of turmeric, has been shown to have both antioxidant and anticataract activity in vitro and in vivo. Green tea also evaluated for anti-cataract potential and it possesses significant antioxidant activity and inhibits oxidative stress-induced experimental cataractogenesis. The aqueous extract of *Ocimum sanctum* protected the cataract in selenite-induced experimental cataractogenesis. A polyherbal preparation, Chyavanprash protects against steroid induced opacities in lens of chick embryo. Many plant extracts are under investigation to exhibit the anti-cataract activity by evaluating them in a various experimental models and estimating the responsible enzymes. However there is no product in the market which is free of side effects and can be effectively used for the treatment of cataract.

### Object of the invention

The object of the present invention is to develop a synergistic herbal composition and its use as a therapeutic agent for delaying the onset and progression of cataract.

### Detailed Description of the Invention:

Accordingly present invention provides a synergistic herbal composition for delaying the progression and onset of cataract comprising the extract of *Foeniculum vulgare, Murraya koenigii* and *Triphala* (mixture of equal amount of *Emblica officinalis, Terminalia chebula* and *Terminalia bellirica)* optionally along with pharmaceutically acceptable excipient.

A synergistic herbal composition of the present invention comprises

| S.No. | Herbs | % age by weight |
|---|---|---|
| 1 | *Foeniculum vulgare* | 0.10 to 2.0 % |
| 2 | *Murraya koenigii* extract | 0.1 to 2.0 % |
| 3 | *Triphala* extract | 0.1 to 5 % |

A synergistic herbal composition of the present invention preferably comprises

| S.No. | Herbs | % age by weight |
|---|---|---|
| 1 | *Foeniculum vulgare* | 0.3 % |
| 2 | *Murraya koenigii* extract | 0.3 % |
| 3 | *Triphala* extract | 0.5 % |

The pharmaceutically acceptable excipient may be selected from the group comprising solubility enhancing agents such as polysorbate, cyclodextrin and their derivative, Cremophore RH 40, viscosity increasing agents such as Hydroxypropylmethylcellulose and other suitable cellulose derivative, polyvinyl alcohol, povidone, carbopol caragenin, antioxidants such as citric acid, EDTA and salts thereof, sodium metabisulphite and other approved water soluble anti oxidants, buffering agents such as citrate ,borate,phosphate,citro- phosphate and osmolarity adjusting agents such as sodium chloride,mannitol,glycerol ,preservatives such as benzalkonium chloride,sorbic acid ,methyl paraben ,propyl paraben, and salts thereof.

The synergistic herbal composition of the present invention is in the form of eye drop, ointment, cream or gel suitable for ophthalmic administration.

The present invention further comprises a process for preparing the said herbal composition. The process of preparing the said herbal composition comprises the following steps:
(a) Coarsely grinding the individual herb such as herein described and extracting it with solvents having different polarity selected from the group such as water, alcohol, acetone, ethyl acetate, Petroleum ether, chloroform or their mixture in different ratio;
(b) Optionally concentrating the extract obtained in step (a) under vacuum and converting the same either in the form of thick paste having around 20-30 % solids or in the form of dry powder;
(c) Formulating pharmaceutically acceptable herbal composition by combining the extracts obtained in step (a) or step (b) in different ratios such as herein described to yield the synergistic herbal composition in the form of eye drop, ointment, cream or gel.

### EXPERIMENTALPROCEDURE:

The invention is illustrated by the following examples. .

In the examples, "part" and "parts" mean "part by weight" and "parts by weight", respectively, unless otherwise specified.

The process for preparing the herbal composition comprises the following steps:
(i) Extraction of herb: The herbs are cleaned, coarsely ground and extracted with water using 8 times the amount of water by heating it to boil for suitable time. The extract is filtered and concentrated to a thick paste having around 30 % of solid content .It is then dried in vacuum drier at a temperature of about 60 C or it can also be spray dried or freeze dried. The dried powder is collected, packed after sieving in double poly bag. Alternatively herbs can be distilled and distillate can be used for further composition development.
(ii) The extracts as per formula are weighed and dissolved in water containing 0.25 % HPMC as viscosity increasing agent under stirring. Additionally Cremophore RH 40 may be added to increase the solubility.
(iii) The preparation in centrifuged at about 4000 rpm for 30-60 min to remove the insoluble matter. Alternatively it can be filtered using whatman filter paper.
(iv) To the clear filtrate suitable preservative (in this case, BKC ,methyl and propyl paraben sodium and sorbic acid, phenyl ethyl alcohol and mercury containing preservatives are found to be suitable) are dissolved.
(v) Disodium EDTA and sodium metabisulphite as anti oxidants are dissolved in it by stirring
(vi) Finally pH is adjusted with boric acid/borax, citric acid, sodium citrate, potassium and sodium phosphate to 5-7 and volume is made up with water for injection.
(vii) The liquid is then filtered using Millipore filter of 0.22 micron and filled in plastic eye drops vial under laminar flow and labeled.
(viii) The final product is clear, transparent having light brown to yellowish red color having osmolarity of 250-300 mosmol and pH of 5-7.

The different herbal compositions were prepared using following formulae:
**(i) *Foeniculum vulgare composition* (F1) comprises:**

| S.N. | Ingredients | Quantity for 100 ml (mg) |
|---|---|---|
| 1 | *Foeniculum vulgare* extract | 300 |
| 2 | Hydroxypropylmethylcellulose | 250 |
| 3 | Methyl paraben sodium | 200 |
| 4 | Propyl paraben sodium | 20 |
| 5 | Disodium EDTA | 100 |
| 6 | Sodium metabisulphite | 100 |
| 7 | Boric acid/Borax quantity sufficient to pH 6.5 | q.s. |
| 8 | Water for injection q.s.to volume | q.s. |

**(ii) *Murraya koenigii* composition (F2) comprises:**

| S.N. | Ingredients | Quantity for 100 ml (mg) |
|---|---|---|
| 1 | *Murraya koenigii* extract | 300 |
| 2 | Hydroxypropylmethylcellulose | 250 |
| 3 | Methyl paraben sodium | 200 |
| 4 | Propyl paraben sodium | 20 |
| 5 | Disodium EDTA | 100 |
| 6 | Sodium metabisulphite | 100 |
| 7 | Boric acid /Borax quantity sufficient to pH 6.5 | q.s. |
| 8 | Water for injection q.s.to volume | q.s. |

**(iii) *Triphala* composition (F3) comprises:**

| S.N. | Ingredients | Quantity for 100 ml (mg) |
|---|---|---|
| 1 | *Triphala* extract | 500 |
| 2 | Hydroxypropylmethylcellulose | 250 |
| 3 | Methyl paraben sodium | 200 |
| 4 | Propyl paraben sodium | 20 |
| 5 | Disodium EDTA | 100 |
| 6 | Sodium metabisulphite | 100 |
| 7 | Boric acid /Borax quantity sufficient to pH 6.5 | q.s. |
| 8 | Water for injection q.s.to volume | q.s. |

*(iv) **Combined Foeniculum vulgare, Murraya koenigii and Triphala composition (F4) of the present invention comprises:***

| S.N. | Ingredients | Quantity for 100 ml (mg) |
|---|---|---|
| 1 | *Foeniculum vulgare* extract | 300 |
| 2 | *Murraya koenigii* extract | 300 |
| 3 | *Triphala* extract | 500 |
| 4 | Hydroxypropylmethylcellulose | 250 |
| 5 | Methyl paraben sodium | 200 |
| 6 | Propyl paraben sodium | 20 |
| 7 | Disodium EDTA | 100 |
| 8 | Sodium metabisulphite | 100 |
| 9 | Boric acid /Borax quantity sufficient to pH 6.5 | q.s. |
| 10 | Water for injection q.s.to volume | q.s. |

Throughout this specification, unless the context requires otherwise, the word "comprise" or variations such as "comprises" or "comprising", will be understood to imply the inclusion of a stated integer or group of integers but not the exclusion of any other integer or group of integers.

### Manufacturing procedure for Composition (F1, F2 and F3):

1. Hydroxypropylmethylcellulose (HPMC) is dissolved in about 50 ml of water under stirring. Once it is dissolved completely, then main active ingredient as per above formula of Composition (F1, F2, and F3) are added to this and dissolved under stirring.
2. The solution is then centrifuged at 4000 rpm for 30 minutes and clear liquid is collected.
3. Methyl and propyl paraben sodium is dissolved in around 10 ml of water and added to clear filtrate of step 2 under stirring.
4. Item number 5 and 6 are then added and dissolved in main bulk under stirring and pH is checked.
5. pH is then adjusted with dilute boric acid/ borax solution and volume is made up.
6. The solution is filtered using 0.22 micron filter aseptically and product is filled in 10 ml three piece plastic vial.

### Manufacturing procedure for Composition (F4):

1. Hydroxypropylmethylcellulose (HPMC) is dissolved in about 50 ml of water under stirring. Once it is dissolved completely, then main active ingredients (item 1, 2 and 3) as per above formula of Composition (F4) are added to this and dissolved under stirring.
2. The solution is then centrifuged at 4000 rpm for 30 minutes and clear liquid is collected.
3. Methyl and propyl paraben sodium is dissolved in around 10 ml of water and added to clear filtrate of step 2 under stirring.
4. Item number 7 and 8 are then added and dissolved in main bulk under stirring and pH is checked.
5. pH is then adjusted with dilute borax/boric acid solution and volume is made up.
6. The solution is filtered using 0.22 micron filter aseptically and product is filled in 10 ml three piece plastic vial.

The activity of herbal composition was evaluated in vivo, in galactose induced cataract model. Wistar rats (50-70g) of either sex were used for the study and fed with 30% galactose.

To evaluate the anti cataract potential of herbal compositions against galactose induced cataract, the rats were divided in to control and treatment groups. A sample was instilled in the form of eye drop (one drop three times a day) starting from one week prior to cataractogenic insult by galactose and was continued till end of the experiment. Instillation of eye drop significantly delayed the onset and the progression of galactose induced cataract as compared to control. The results of these experimental trials are provided below.

### Evaluation of Anti-Cataract Potential:

**Table 1: Effect of 0.5% Triphala eye drops in galactose induced cataractogenesis of rat.**

| **Days** | **Stages** | **Control (n=20)** | **0.5% Eye Drops (n=20)** |
|---|---|---|---|
| 7^{th} Day | Normal | ------ | 85% |
| | Stage-I | 100% | 15% |
| | Stage-II | ------- | ------ |
| | Stage-III | ---------- | ------- |
| | Stage-IV | -------- | -------- |
| | **Opacity Index** | **1** | **0.16 **** |
| 14^{th} Day | Normal | ---- | 10% |
| | Stage-I | ----- | 80% |
| | Stage-II | 100% | 10% |
| | Stage-III | ----- | ----- |
| | Stage-IV | ----- | ----- |
| | **Opacity Index** | **2** | **1.1 **** |
| 21^{st} Day | Normal | ----- | ----- |
| | Stage-I | ----- | 80% |
| | Stage-II | ----- | ---- |
| | Stage-III | 85% | 10% |
| | Stage-I | 15% | 10% |
| | **Opacity Index** | **3.15** | **1.7 **** |
| 30^{th} Day | Normal | ----- | ----- |
| | Stage-I | ----- | 50% |
| | Stage-II | ----- | 20% |
| | Stage-III | ----- | ----- |
| | Stage-IV | 100% | 30% |
| | **Opacity Index** | **4** | **2.3 **** |

| | | | |
|---|---|---|---|
| **P<0.01 as compared to control. | | | |

**Table 2: Effect of 0.3% eye drops of Foeniculum vulgare in galactose induced cataractogenesis of rat.**

| **Days** | **Stages** | **Control (n=20)** | **0.3% eye drops (n=20)** |
|---|---|---|---|
| 7^{th} Day | Normal | ---- | 70% (14) |
| | Stage I | 100% (20) | 30% (6) |
| | Stage II | ---- | ---- |
| | Stage III | ---- | ---- |
| | **Opacity Index** | **1** | **0.3 **** |
| 14^{th} Day | Normal | ---- | 10% (2) |
| | Stage I | 15% (3) | 75% (15) |
| | Stage II | 85% (17) | 15% (3) |
| | Stage III | ---- | ---- |
| | **Opacity Index** | **1.85** | **1.05 **** |
| 21^{st} Day | Normal | ---- | ---- |
| | Stage I | ---- | 55% (11) |
| | Stage II | ---- | 25% (5) |
| | Stage III | 100% (20) | 20% (4) |
| | Stage IV | ---- | ---- |
| | **Opacity Index** | **3** | **1.65 **** |
| 30^{th} Day | Normal | ---- | ---- |
| | Stage I | ---- | 25% (5) |
| | Stage II | ---- | 40% (8) |
| | Stage III | 5% | 20% (4) |
| | Stage IV | 95% (9) | 15% (3) |
| | **Opacity Index** | **3.95** | **2.25 **** |

| | | | |
|---|---|---|---|
| *P<0.05 as compared to control. **P<0.01 as compared to control. | | | |

**Table 3: Effect of Murraya koenigii(MK) 0.3% eye drops in galactose induced cataractogenesis of rat.**

| **Days** | **Stages** | **Control (n=16)** | **MK (n=14)** |
|---|---|---|---|
| 7^{th} Day | Normal | ---- | 50% (07) |
| | Stage-I | 100% (16) | 50% (07) |
| | Stage-II | ---- | ---- |
| | Stage-III | ---- | ---- |
| | Stage-IV | ---- | ---- |
| | **Opacity Index** | **1** | **0.350 **** |
| 14^{th} Day | Normal | ---- | 42.8% (06) |
| | Stage-I | 18.75% (3) | 57.2% (08) |
| | Stage-II | 81.25% (13) | ---- |
| | Stage-III | ----- | ---- |
| | Stage-IV | ----- | ---- |
| | **Opacity Index** | **1.812** | **0.571** ** |
| 21^{st} Day | Normal | ----- | 22.4% (3) |
| | Stage-I | ----- | 42.2% (6) |
| | Stage-II | 25% (4) | 35.4% (5) |
| | Stage-III | 75% (12) | ----- |
| | Stage-IV | ----- | ----- |
| | **Opacity Index** | **2.750** | **1.142 **** |
| 30^{th} Day | Normal | ---- | ---- |
| | Stage-I | ---- | 28.0% (4) |
| | Stage-II | ---- | 7.60% (1) |
| | Stage-III | 6.25% (1) | 57.40% (8) |
| | Stage-IV | 93.75% (15) | 7.0% (1) |
| | **Opacity Index** | **3.937** | **2.142**** |

| | | | |
|---|---|---|---|
| **P<0.01 as compared to control. | | | |

**Table 4: Effect of composition F4 comprising of Triphala 0.5%, Murraya koenigii 0.3% and Foeniculum vulgare 0.3%) eye drops in galactose induced cataractogenesis of rat.**

| **Days** | **Stages** | **Control (n=16)** | **Combination (n=14)** |
|---|---|---|---|
| 7^{th} Day | Normal | ---- | 78.5% (11) |
| | Stage-I | 100% (16) | 21.5% (03) |
| | Stage-II | ---- | ---- |
| | Stage-III | ---- | ---- |
| | Stage-IV | ---- | ---- |
| | **Opacity Index** | **1** | **0.214**** |
| 14^{th} Day | Normal | ---- | 64.20% (9) |
| | Stage-I | 18.75% (3) | 35.80% (5) |
| | Stage-II | 81.25% (13) | ---- |
| | Stage-III | ----- | ---- |
| | Stage-IV | ----- | ---- |
| | **Opacity Index** | **1.812** | **0.357 **** |
| 21^{st} Day | Normal | ----- | 57.1% (8) |
| | Stage-I | ----- | 42.9% (6) |
| | Stage-II | 25% (4) | ---- |
| | Stage-III | 75% (12) | ----- |
| | Stage-IV | ----- | ----- |
| | **Opacity Index** | **2.750** | **0.428**** |
| 30^{th} Day | Normal | ----- | 14.4% (2) |
| | Stage-I | ----- | 50.3% (7) |
| | Stage-II | ----- | 28.3% (4) |
| | Stage-III | 6.25% (1) | 7.0% (1) |
| | Stage-IV | 93.75% (15) | ---- |
| | **Opacity Index** | **3.937** | **1.285 **** |

| | | | |
|---|---|---|---|
| **P<0.01 as compared to control. | | | |

### Comparative results of Composition of individual herbal ingredients (F1, F2 ,F3) and combination composition (F4) in terms of reducing opacity of lens.

**Table 5:Opacity index of different composition at different period of time**

| Day | Control | *Triphala* eye drops (0.5%)(F3) | F.vulgare eye drops (0.3%) (F1) | M.koeingii eye drops(0.3%)(F2) | Combination composition(F4) |
|---|---|---|---|---|---|
| 7 | 1 | 0.16 | 0.30 | 0.35 | 0.21 |
| 14 | 2 | 1.10 | 1.05 | 0.57 | 0.36 |
| 21 | 3.15 | 1.70 | 1.65 | 1.14 | 0.43 |
| 30 | 4 | 2.30 | 2.25 | 2.14 | 1.28 |

Surprisingly the herbal composition of the present invention comprises *Foeniculum **vulgare,** Murraya koenigii, and Triphala* (F4) along with pharmaceutically acceptable carriers or excipient showed enhanced effects as compared to the individual compositions (F1, F2 and F3). The results clearly indicates that the combination composition has improved opacity of lens and also prevented occurrence of cataract of higher degree in comparison to their individual composition therefore the said herbal composition (F4) is synergistic.

**Table 6: Effect of Composition (F4) on 30 mM Galactose induced osmotic stress in rat lenses: In vitro study.**

| Group (n=6) | **GSH** µmole/g. of tissue | **Polyols** µg/mg of tissue |
|---|---|---|
| Normal | 1.49±0.05 * | 0.05±0.002 * |
| Control | 0.16 | 1.10 |
| 55µg/ml | 1.47±0.07 * | 0.24±0.01* |
| 110µg/ml | 1.10±0.07* | 0.84±0.07 * |
| 220µ/ml | 0.91±0.06 * | 0.97±0.04 ns |

| | | |
|---|---|---|
| * P < 0.01, and ns- non significant as compared with control | | |

**Table 7: Effect of Composition (F4) on 100µm sodium selenite induced oxidative stress in rat lenses: In vitro study.**

| Group (n=6) | **GSH** µmole/g. of tissue | **MDA** µmole/g. of tissue |
|---|---|---|
| Normal | 1.22±0.09 * | 2.80±0.09 * |
| Control | 0.13 | 26.65 |
| 55µg/ml | 1.11±0.07 * | 3.29±0.18* |
| 110µg/ml | 0.87±0.03 * | 6.26±0.24 * |
| 220µg/ml | 0.50±0.03 * | 8.44±0.40 * |

| | | |
|---|---|---|
| * P < 0.01 as compared with control. | | |

The composition was studied in galactose induced osmotic stress *in vitro* and various bio chemical parameters were estimated to assess the mechanism of action. The composition was also evaluated in sodium selenite induced cataract in rat pups.

## Claims

1. A synergistic herbal composition in the form of eyedrop, ointment, cream or gel suitable for ophthalmic administration for use in delaying the progression and onset of cataract comprising aqueous extract of herbs *Foeniculum **vulgare,** Murraya koenigii* and *Triphala* (mixture of equal amounts of *Emblica officinalis, Terminalia chebula and Terminalia bellirica*) optionally along with a pharmaceutical acceptable excipient, wherein the composition comprises
| S.No. | Herbs | %age by weight |
|---|---|---|
| 1 | *Foeniculum vulgare* | 0.10 to 2.0 % |
| 2 | *Murraya koenigii* extract | 0.1 to 2.0 % |
| 3 | *Triphala* extract | 0.1 to 5 % |

2. A synergistic herbal composition as claimed in claim 1, wherein the composition comprises
| S_{.}No. | Herbs | %age by weight |
|---|---|---|
| 1 | *Foeniculum vulgare* | 0.3 % |
| 2 | *Murraya koenigii* extract | 0.3 % |
| 3 | *Triphala* extract | 0.5 % |

3. A synergistic herbal composition as claimed in claim 1, wherein the pharmaceutically acceptable excipient is selected from the group comprising a solubility enhancing agent such as polysorbate, cyclodextrin and their derivative, polyoxyl 40 hydrogenated castor oil, Viscosity increasing agents such as Hydroxypropylmethylcellulose, polyvinyl alcohol, povidone, carbopol, caragenin, anti oxidants such as citric add, EDTA and salts thereof, or sodium metabisulphite, buffering agents such as citrate ,borate, phosphate, citro phosphate and osmolarity adjusting agents such as sodium chloride, mannitol, glycerol, preservatives such as benzalkonium chloride, sorbic acid, methyl paraben, propyl paraben and salts thereof.

4. A process for preparing a synergistic herbal composition in the form of eye drop, ointment, cream or gel suitable for ophthalmic administration for use in delaying the progression and onset of cataract comprising extract of herbs *Foeniculum **vulgare,** Murraya koenigii* and *Triphala* (mixture of equal amount of *Emblica officinalis, Terminalia chebula and Terminalia bellirica*) wherein the said process comprises the following steps;
(a) Coarsely grinding the individual herb and extracting it with water;
(b) Optionally concentrating the extract obtained in step (a) under vacuum and converting the same either in the form of thick paste having around 20-30 % solids or in the form of dry powder;
(c) Formulating pharmaceutically acceptable herbal composition by combining the extracts obtained in step (a) or step (b) in different ratios to yield the synergistic herbal composition in the form of eye drop, ointment, cream or gel, wherein the composition comprises
| S.No. | Herbs | %age by weight |
|---|---|---|
| 1 | *Foeniculum vulgare* | 0.10 to 2.0 % |
| 2 | *Murraya koenigii* extract | 0.1 to 2.0 % |
| 3 | *Triphala* extract | 0.1 to 5 % |

## Patentansprüche

1. Eine synergistische pflanzliche Zusammensetzung in Form von Augentropfen, Salbe, Creme oder Gel, die zur ophthalmischen Anwendung geeignet ist zur Verwendung in der Verzögerung des Fortschritts und des Einsetzens eines Katarakts, die wässrige Extrakte der Kräuter *Foeniculum vulgare, Murraya **koenigii*** und *Triphala* (eine Mischung gleicher Mengen von *Emblica officinalis, Terminalia chebula* und *Terminalia bellirica*) ggf. zusammen mit einem pharmazeutisch annehmbaren Hilfsstoff aufweist, wobei die Zusammensetzung Folgendes aufweist:
| S.Nr. | Kräuter | Gew.-% |
|---|---|---|
| 1 | *Foeniculum vulgare* | 0,10 bis 2,0 Gew.-% |
| 2 | *Murraya koenigii E*xtrakt | 0,1 bis 2,0 Gew.-% |
| 3 | *Triphala* Extrakt | 0,1 bis 5 Gew.-% |

2. Eine synergistische pflanzliche Zusammensetzung wie in Anspruch 1 beansprucht, wobei die Zusammensetzung Folgendes aufweist:
| S.Nr. | Kräuter | Gew.-% |
|---|---|---|
| 1 | *Foeniculum vulgare* | 0,3 Gew.-% |
| 2 | *Murraya koenigii* Extrakt | 0,3 Gew.-% |
| 3 | *Triphala* Extrakt | 0,5 Gew.-% |

3. Eine synergistische pflanzliche Zusammensetzung wie in Anspruch 1 beansprucht, wobei der pharmazeutisch annehmbare Hilfsstoff ausgewählt ist aus der Gruppe bestehend aus einem die Löslichkeit erhöhenden Mittel, wie beispielsweise Polysorbat, Cyclodextrin und dessen Derivate, Polyoxyl 40 hydriertes Rizinusöl, den die Viskosität erhöhenden Mittel wie beispielsweise Hydroxypropylmethylzellulose, Polyvinylalkohol, Povidon, Karbopol, Karrageen, den Antioxidanzien wie beispielsweise Zitronensäure, EDTA und Salze davon oder Natriummetabisulfit, den Puffermitteln wie beispielsweise Zitrat, Borat, Phosphat, Zitrophosphat und den die Osmolarität einstellenden Mitteln wie beispielsweise Natriumchlorid, Mannitol, Glyzerin, den Konservierungsmitteln wie beispielsweise Benzalkoniumchlorid, Sorbinsäure, Methylparaben, Propylparaben und Salze davon.

4. Verfahren zum Herstellen einer synergistischen pflanzlichen Zusammensetzung in Form von Augentropfen, Salbe, Creme oder Gel, die für die ophthalmische Anwendung geeignet ist zur Verwendung in der Verzögerung des Fortschritts und des Einsetzens eines Katarakts, die Extrakte der Kräuter *Foeniculum vulgare, Murraya koenigii* und *Triphala* (eine Mischung gleicher Mengen von *Emblica officinalis, Terminalia chebula* und *Termnalia bellirica*) aufweist, wobei das Verfahren die folgenden Schritte aufweist;
(a) Grobes Mahlen der einzelnen Kräuter und Extrahieren mit Wasser;
(b) ggf. Konzentrieren des im Schritt (a) erhaltenen Extrakts unter Vakuum und Konvertieren desselben in die Form einer dicken Paste, die etwa 20-30 % Feststoff aufweist oder in die Form eines trockenen Pulvers;
(c) Formulieren einer pharmazeutisch annehmbaren pflanzlichen Zusammensetzung durch Kombinieren der in Schritt (a) oder Schritt (b) erhaltenen Extrakte in verschiedenen Verhältnissen, um die synergistische pflanzliche Zusammensetzung in Form von Augentropfen, Salbe, Creme oder Gel zu erhalten, wobei die Zusammensetzung Folgendes aufweist
| S.Nr. | Kräuter | Gew.-% |
|---|---|---|
| 1 | *Foeniculum vulgare* | 0,10 bis 2,0 Gew.-% |
| 2 | *Murraya koenigii E*xtrakt | 0,1 bis 2,0 Gew.-% |
| 3 | *Triphala* Extrakt | 0,1 bis 5 Gew.-% |

## Revendications

1. Composition végétale synergique sous forme de collyre, d'onguent, de crème ou de gel, adaptée à une administration ophtalmique pour emploi dans le retard de la progression et de l'apparition de la cataracte, et comprenant un extrait aqueux des végétaux *Foeniculum vulgare, Murraya koenigii* et *Triphala* (mélange à parts égales *d'Emblica officinalis,* de *Terminalia chebula* et de *Terminalia bellirica*) éventuellement accompagné d'un excipient de qualité pharmaceutique, la composition comprenant
| S. n° | Végétal | Pourcentage massique |
|---|---|---|
| 1 | *Foeniculum vulgare* | 0,10 à 2,0 % |
| 2 | *extrait de Murraya koenigii* | 0,1 à 2,0 % |
| 3 | extrait de *Triphala* | 0,1 à 5 % |

2. Composition végétale synergique conforme à la revendication 1, où la composition comprend
| S. n° | Végétal | Pourcentage massique |
|---|---|---|
| 1 | *Foeniculum vulgare* | 0,3 % |
| 2 | extrait de *Murraya koenigii* | 0,3 % |
| 3 | extrait de *Triphala* | 0,5 % |

3. Composition végétale synergique conforme à la revendication 1, où l'excipient de qualité pharmaceutique est choisi dans le groupe constitué des suivants : un agent de solubilisation tel que polysorbate, cyclodextrine et ses dérivés, huile de ricin hydrogénée polyoxyl 40, des agents d'augmentation de viscosité tels qu'hydroxypropylméthylcellulose, alcool polyvinylique, povidone, carbopol, carraghénine, des antioxydants tels qu'acide citrique, EDTA et ses sels ou métabisulfite de sodium, des agents tampons tels que citrate, borate, phosphate, citrophosphate, des agents d'ajustement de l'osmolarité tels que chlorure de sodium, mannitol, glycérol, des conservateurs tels que chlorure de benzalkonium, acide sorbique, méthylparabène, propylparabène et leurs sels.

4. Procédé d'élaboration d'une composition végétale synergique sous forme de collyre, d'onguent, de crème ou de gel, adaptée à une administration ophtalmique pour emploi dans le retard de la progression et de l'apparition de la cataracte, et comprenant un extrait aqueux des végétaux *Foeniculum vulgare, Murraya koenigii* et *Triphala* (mélange à parts égales *d'Emblica officinalis,* de *Terminalia chebula* et de *Terminalia bellirica*), ledit procédé comprenant les étapes suivantes :
(a) Broyage grossier des végétaux individuels et leur extraction à l'eau ;
(b) Éventuellement concentration de l'extrait obtenu à l'étape (a) sous vide et sa conversion soit en une pâte épaisse de proportion en matière sèche comprise entre environ 20 et 30 %, soit en une poudre sèche ;
(c) Formulation de la composition végétale de qualité pharmaceutique en combinant les extraits obtenus à l'étape (a) ou à l'étape (b) en divers rapports pour obtenir la composition végétale synergique sous forme de collyre, d'onguent, de crème ou de gel, la composition comprenant
| S. n° | Végétal | Pourcentage massique |
|---|---|---|
| 1 | *Foeniculum vulgare* | 0,10 à 2,0 % |
| 2 | extrait de *Murraya koenigii* | 0,1 à 2,0 % |
| 3 | extrait de *Triphala* | 0,1 à 5 % |
